# EUROPEAN PATENT APPLICATION

(11) **EP 4 799 620 A1**
(43) Date of publication of application: **02.09.2026**
(21) Application number: 24895244.2
(22) Date of filing: 22.11.2024
(51) Int. Cl.: A61K 31/4196, A61K 31/496, A61K 9/14, A61K 9/16, A61P 25/24, A61P 43/00

(54) **ORAL PHARMACEUTICAL FORM FOR BIPHASIC RELEASE, COMPOSED OF A MULTIPARTICULATE SYSTEM, PROCESS FOR PREPARING SAME AND THERAPEUTIC USE OF ORAL FORM**

(30) Priority: 30.11.2023 BR 102023025187
(71) Applicant: Prati, Donaduzzi & Cia LTDA, 85903-630 Toledo (BR)
(72) Inventor: BRUM JUNIOR, Liberato, 85901-110 Toledo-PR (BR); WEBLER, Emanuelle, 85902-260 Toledo-PR (BR); RECHIA, Leticia Mello, 85905-620 Toledo-PR (BR); TONDO FILHO, Volnei José, 85900-050 Toledo-PR (BR); GOZZI, Paula Thais, 85911-150 Toledo-PR (BR); ZIMMERMANN, Patrícia Moura da Rosa, 85905-590 Toledo-PR (BR); DE SOUZA, Fabio Pinheiro, 85903-470 Toledo-PR (BR); DE ANDRADE, Elvis Pimenta, 85904-450 Toledo-PR (BR); ILIPRONTI, Thiago, 85914-140v Toledo-PR (BR); LICKOWSKI, Dirceu, 85905-530 Toledo-PR (BR); DONADUZZI, Carmen Maria, 85903-180 Toledo-PR (BR)
(74) Representative: Linage González, Rafael
(86) International application number: PCT/BR2024/050536
(87) International publication number: WO 2025/111679

(57) **Abstract**

The present invention relates to an oral pharmaceutical form comprising a core, a coating layer comprising the drug, a modified-release layer, an immediate-release layer comprising the active ingredient, and a polymeric layer, which refers to the outermost layer. Further, a slider will be deposited on this layer, which will remain on the surface of the pharmaceutical form. The invention provides, in a single pharmaceutical form, a biphasic release system that promotes the release of the active pharmaceutical ingredient in two stages. In addition, the invention relates to a process for manufacturing the aforementioned pharmaceutical form, as well as to the therapeutic use thereof.

## Description

### FIELD OF THE INVENTION

The present invention relates to a pharmaceutical form comprising an inert core, a coating layer comprising the active pharmaceutical ingredient (API) trazodone, a modified-release layer, an immediate-release layer comprising the API, and a polymeric layer, which refers to the outermost layer. Further, a slider will be deposited on this layer, which will remain on the surface of the pharmaceutical form, eliminating the static energy of the microgranules and providing desirable differentials in the production process.

The present invention further relates to a controlled drug delivery system capable of promoting the biphasic release of trazodone. Said controlled drug release system is also capable of promoting the biphasic release of other compounds such as, for example, mirtazapine, cannabidiol, zolpidem, zopiclone, cyclobenzaprine, quetiapine, clonazepam, among others.

In addition, the invention relates to a process for manufacturing the aforementioned pharmaceutical form, as well as to the therapeutic use thereof.

The invention relates to multiparticulate systems containing the drug subdivided into functional units, which may be pellets, granules, or minitablets. Said systems offer technological and biopharmacotechnical advantages when compared to monolithic systems, while also providing therapeutic benefits for patients. Therefore, they have increasingly stood out among the new pharmaceutical forms and drug delivery systems.

### BACKGROUND OF THE INVENTION

Trazodone is a triazolopyridine derivative that belongs to the class of serotonin receptor type 2 (5-HT2) antagonists and serotonin reuptake inhibitors (SERT). Although its mechanism of action is not fully understood, the primary pharmacological action of trazodone is the blockade of the 5-hydroxytryptamine 2A receptor (5-HT2a). Higher doses of trazodone cause antagonism at H1 histaminergic and α1-adrenergic receptors. It is believed that the blockade of 5-HT2A, histamine H1, and alpha receptors produces the hypnotic effect reported for low doses of trazodone. At low doses, trazodone induces and maintains sleep without causing daytime drowsiness or tolerance, mainly due to its short half-life (3 to 6 hours). For the antidepressant effects of the medication to achieve efficacy, the simultaneous blockade of 5-HT2A and SERT is necessary, which occurs at higher doses (150 to 600 mg). Tolerance may occur during the combined antagonistic actions of 5-HT2A and SERT.

Trazodone is a psychoactive compound with sedative and antidepressant properties that is rapidly absorbed by the upper gastrointestinal tract and extensively metabolized after oral administration. Normally, trazodone is used to relieve symptoms of depression, such as feelings of sadness, worthlessness, or guilt; loss of interest in daily activities; changes in appetite; fatigue; thoughts of death or suicide.

Trazodone is normally used in its hydrochloride form as Formula I below. The preparation of this compound was first disclosed in U.S. Patent US 3,381,009,

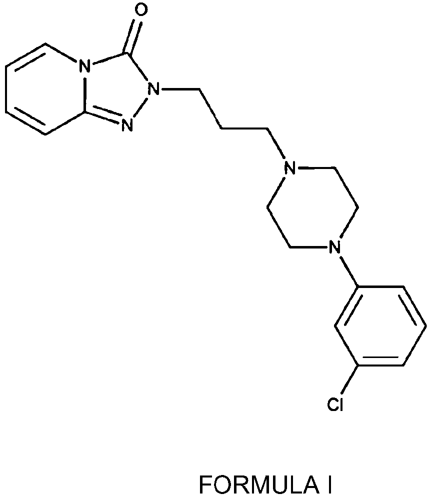

The solubility of trazodone depends on the pH and has a pKa of 6.74 in water. As a result, trazodone is highly soluble in acidic medium (as found in the stomach and upper intestine), that is, when the pH of the medium is below its pKa. In contrast, when above its pKa, its solubility is very low, for example, under the neutral and basic conditions of the large intestine. Such insolubility obviously has an effect on its dissolution and, therefore, on the availability of the active pharmaceutical ingredient for absorption in the large intestine. Therefore, it would be expected that these characteristics would make it difficult to develop extended-release forms of trazodone (for example, more than 8 hours), which require substantially uniform absorption throughout the gastrointestinal tract, in particular, absorption during passage through both the upper and lower intestinal tracts.

As with many medications, trazodone is normally prescribed as an immediate-release form for use twice (BID) or three times (TID) a day, with all the drawbacks and disadvantages that this entails. For example, multiple daily doses may result in concentrations of the active pharmaceutical ingredient in the blood that do not remain within the so-called therapeutic range and that, therefore, may be associated with greater risks of dose-related adverse effects when reaching high levels, or a lower degree of efficacy when reaching low levels.

As a result, there is a need for new trazodone delivery systems capable of making this active ingredient available more effectively, promoting optimization of the distribution of the API throughout the body, avoiding undesirable fluctuations in its concentration and, thus, influencing the duration of the drug's action and side effects resulting from its use.

The document US 2006/172006 refers to a composition in the form of a controlled-release tablet of tramadol hydrochloride that provides an analgesic effect within 2 hours after oral administration and for at least 24 hours after administration. A 200 mg dose of this controlled-release composition of the invention would provide a rapid onset of analgesic effect within 2 hours after oral administration and a mean plasma concentration of tramadol between 100 ng/mL and 200 ng/mL for at least 24 hours after a single dose. The tablet core of said document would comprise at least one active ingredient and a matrix, and these components would be associated such that release of the pharmaceutical ingredient from the matrix would be controlled. In one embodiment of the invention, the core matrix would be a crosslinked starch with a high amylose content (Contramid ^{©}). The composition of the core may further include binders, lubricants, disintegrating agents, and pharmaceutically acceptable carriers.

The Penumatcha, Kiran, 2003¹ document refers to the preparation of tablets comprising a controlled-release trazodone hydrochloride matrix, in which trazodone is dispersed in a hydroxypropyl methylcellulose (HPMC) matrix. The tablet further comprises excipients such as microcrystalline cellulose (Avicel^{©}), magnesium stearate, and colloidal silicon dioxide (Cab-O-Sil^{©}). Said controlled-release tablets would provide desired plasma levels within the therapeutic range over a long period of time (24 hours).

The document BR PI 0615860-9 discloses a solid monolithic extended-release pharmaceutical composition in the form of a tablet that, when subdivided into smaller pharmaceutical forms, would present substantially the same drug release profile as the composition from which it was derived. The solid extended-release composition of said document would comprise an extended-release matrix including a controlled-release excipient, preferably a high-amylose cross-linked starch such as Contramid ^{©}, and an active pharmaceutical ingredient disposed in the matrix, trazodone, wherein the composition releases this active ingredient for at least 24 hours and wherein the composition has a hardness of 100 N to 350 N. The composition further comprising a binding agent, a solubilizing agent, an acidifying agent, a pore-forming agent, a lubricant, and a slider.

As can be observed, different oral pharmaceutical forms have been developed to control the release profile of trazodone, particularly pharmaceutical forms in tablet form using controlled-release strategies of the active pharmaceutical ingredient to achieve stable and effective concentrations for 24 hours or more.

It is based on this scenario that the present invention arises, which provides a stable pharmaceutical form and a biphasic release system that promotes the release of the active pharmaceutical ingredient trazodone in two steps to maintain therapeutic levels, particularly for the treatment of sleep disorders, minimizing nighttime awakenings and improving sleep onset and maintenance throughout the night.

It is further emphasized that the proposed API release system is innovative because it combines, in a single pharmaceutical form, a core, a coating layer of the active ingredient, a modified-release layer, and an immediate-release layer of the active in order to release the active pharmaceutical ingredient at different times in two immediate-release peaks of the active ingredient, with the first release peak occurring within up to two hours after administration and the second peak occurring between four and seven hours after administration. On the immediate-release layer, a polymeric film is also deposited, and on this outermost layer a slider will be added, which will remain on the surface of the pharmaceutical form.

In this way, the invention provides a novel drug delivery system that encompasses the desired therapeutic effect for inducing and maintaining sleep throughout the night through a biphasic release technology, which consists of two immediate drug release peaks that behave as an extended release, favoring sleep induction after ingestion of the medication and promoting sleep maintenance, which is sustained by the second peak of active ingredient release.

Currently, trazodone is indicated for the treatment of depression, and to obtain antidepressant effects, the simultaneous blockade of 5-HT2A and serotonin reuptake is necessary, which occurs at higher doses (150-600 mg).

Increasing the dose of trazodone causes antagonism at histamine H1 and α1 adrenergic receptors. However, trazodone has moderate antihistaminic activity and low anticholinergic activity, and it moderates suppression of cortisol in the hypothalamic-pituitary-adrenal axis, which likely contributes to its efficacy in insomnia. It is believed that the blockade of 5-HT2A, histamine H1, and alpha receptors produces the hypnotic effect reported for low doses of trazodone (25-100 mg), in which it induces and maintains sleep without causing daytime drowsiness (Jaffer et al, 2017).

Data described in the scientific literature indicate that trazodone may have the potential to be effective and safe in improving sleep quality.

Sedative effects of trazodone at doses of 50 mg/day for the treatment of insomnia were evaluated in 67 pregnant women, with reports of efficacy during the third trimester of pregnancy (Khazaie et al, 2013). The 50 mg/day dose was also effective in patients with Alzheimer's disease (Camargos et al, 2014), and was also effective in maintaining sleep in a double-blind controlled study (Roth et al, 2011).

Double-blind controlled study in patients with Alzheimer's disease proved that the 50 mg/day dose was safe and effective in treating insomnia in this patient population (Camargos et al, 2014). Another cohort study of postmenopausal women reported that both zopiclone and trazodone (50-100 mg/day) improved sleep quality and sexual dysfunction (Eraslan et al, 2014).

Doses of 25-150 mg/day have been shown to improve sleep quality in patients with primary insomnia with a mean age of 46 years (Wichniak et al, 2007).

Trazodone increased slow waves during sleep in healthy volunteers at daily doses of 50 and 100 mg (Yamadera et al, 1999). Improvement in sleep quality was also observed in a study with daily doses of 50-100 mg for the treatment of antidepressant-induced secondary insomnia (Nierenberg et al, 1994).

In short, the proposal to develop formulations with biphasic release is based on the premise that trazodone has effects on sleep quality at lower doses than those indicated for the treatment of depression.

As previously reported, there are reports in literature of its antihistaminic activity and low anticholinergic activity, and it is believed that the blockade of 5-H2A, histamine H1, and alpha receptors produces the hypnotic effect at doses between 25-100 mg.

Based on the foregoing, a double-blind clinical trial was conducted to evaluate the impact of the medication on sleep quality and total sleep time, as well as to quantify the reduction in sleep latency in patients diagnosed with comorbid insomnia, through actigraphy and assessment scales.

In order to demonstrate the effectiveness of the developed formulation, a RANDOMIZED, CONTROLLED, CROSS-OVER CLINICAL TRIAL WAS CONDUCTED TO EVALUATE SLEEP QUALITY IN PATIENTS WITH SECONDARY INSOMNIA TREATED WITH TRAZODONE OR PLACEBO. The objective of the clinical trial was to evaluate the impact of the medication on sleep quality and total sleep time, as well as to quantify the reduction in sleep latency in patients diagnosed with comorbid insomnia, through actigraphy and assessment scales. The study was conducted with 30 patients.

The results of the analyses of the questionnaires, as well as the subjective perception of sleep latency, total sleep time, sleep efficiency, and awakenings, are shown in the table below.

**TABLE 1: RESULTS OF THE ANALYSIS OF THE QUESTIONNAIRES AND OF THE SUBJECTIVE SLEEP PERCEPTION.**

| | Consultation 1 | Consultation 2 | Consultation 3 | Consultation 4 | p |
|---|---|---|---|---|---|
| Hamilton Depression Scale | 17,21±9,82@# | 13.21±6.40&* | 11,74±6,80#& | 7.96±3.93@* | **<0,001** |
| Hamilton Depression Scale 50 mg | 16.31±8.60@# | 11.88±6.06@ | 11.13±6.98 | 7,875±4.28# | **0,002** |
| Hamilton Depression Scale 75 mg | 18.31±11.41@ | 14.85±6.65# | 12.5±6.81* | 8.08±3.57@# | **0,004** |
| Pittsburgh Sleep Quality Questionnaire | 13.86±3.21@ | 11.9±2.69# | 12.59±3.68* | 9.85±3.34@# | **0,002** |
| Pittsburgh Sleep Quality Questionnaire 50 mg | 14.13±3.11@# | 11.19±2.90# | 11.80±4.02 | 9.81±3.50@ | **0,002** |
| Pittsburgh Sleep Quality Questionnaire 75 mg | 13.54±3.43@ | 12.77±2.20 | 13.58±3.08# | 9.91±3.26@# | **0,006** |
| Athens Insomnia Index | 16.54±4.32@# | 15.07±4.77&* | 10.48±6.78#& | 5.75±5.26@* | **<0,001** |
| Athens Insomnia Index 50 mg | 16.4±4.88@# | 13.81±5.36&* | 8.86±6.30#& | 5.81±5.57@* | **<0,001** |
| Athens Insomnia Index 75 mg | 16.69±3.77@ | 16.62±3.52#* | 12.5±7.09* | 5.66±5.05@# | **<0,001** |
| Insomnia Severity Scale | 20.41±3.93@# | 18.57±5.32&* | 13.81±8.14#& | 7.42±4.90@# | **<0,001** |
| Insomnia Severity Scale 50 mg | 19.88±4.04@# | 17.69±6.18&* | 11.14±7.24#& | 7.50±4.48@# | **<0,001** |
| Insomnia Severity Scale 75 mg | 21.08±3.84@ | 19.75±3.84# | 16.69±8.34 | 7,33±5,61@# | **<0,001** |
| Sleep latency (min.) | 69,59±74,51@ | 51.76±36.18# | 51.76±50.13 | 29,69±25,24@# | **<0,001** |
| Sleep latency (min.) 50mg | 50.5±40.46 | 37.19±20.00 | 42,56±47,20 | 26.31±25.20 | 0,060 |
| Sleep latency (min.) 75mg | 93.08±99.11 | 69.69±43.86 | 63.08±53.17 | 33.85±25.67 | 0,070 |
| Total sleep time (min.) | 277.20±109.20@ | 291.70±81.33 | 322.80±94.07 | 341.80±110.00@ | **0,040** |
| Total sleep time (min.) 50mg | 273.80±95.42 | 307.50±75.50 | 348.80±103.30 | 312.00±118.50 | 0,110 |
| Total sleep time (min.) 75mg | 281.50±128.20 | 272.30±87.00# | 290.80±72.85 | 378.50±89.61# | **0,010** |
| Nights with awakenings/week | 2.93±0.37@ | 2.67±0.82 | 2.26±1.08# | 1.20±1.45@# | **<0,001** |
| Nights with awakenings/week 50 mg | 3.00±0.00@ | 2.40±1.05 | 2.00±1.17# | 1.06±1.39@# | **<0,001** |
| Nights with awakenings/week 75 mg | 2.84±0.55 | 3.00±0.00# | 2.58±0.90 | 1.38±1.55# | **0,033** |

The use of Trazodone improved scores on the Hamilton Depression Scale, the Pittsburgh Sleep Quality Questionnaire, the Athens Insomnia Index, the Insomnia Severity Scale, sleep latency, total sleep time, and weekdays with awakenings.

Of the 29 patients, 24 correctly completed the proposed actigraphy usage period. The results are in Table 2.

**TABLE 2: ACTIGRAPHY ANALYSIS RESULTS.**

| | Actigraphy 1 | Actigraphy 2 | Actigraphy 3 | p |
|---|---|---|---|---|
| Sleep latency (min.) | 6.37±5.60 | 6.47±6.51 | 5.50±5.97 | 0,600 |
| Sleep latency (min.) 50mg | 6.13±5.56 | 7.16±7.48 | 4.40±4.40 | 0,200 |
| Sleep latency (min.) 75mg | 6.77±5.96 | 5.28±4.71 | 7.33±7.90 | 0,150 |
| Total sleep time (min.) | 470.90±122.70 | 491.90±121.50 | 484.50±104.00 | 0,130 |
| Total sleep time (min.) | 50mg 461.80±106.60 | 479.90±95.25 | 476.00±91.94 | 0,600 |
| Total sleep time (min.) | 75 mg 486.00±151.70 | 514.10±166.50 | 498.60±126.30 | 0,120 |
| Sleep efficiency (%) | 83.07±4.75 @ | 84.27±4.34 | 84.86±4.39 @ | **0,030** |
| Sleep efficiency (%) 50 mg | 83.04±4.25 @ | 84.77±4.97 | 85.41±4.56 @ | **0,020** |
| Sleep efficiency (%) 75 mg | 83.1±5.77 | 83.35±2.97 | 83.93±4.19 | 0,600 |
| WASO (min.) | 84.20±26.09 | 75.38±29.34 | 73.00±26.45 | 0,110 |
| WASO (min.) 50mg | 84.20±26.09 | 75.38±29.34 | 73±26.45 | 0,110 |
| WASO (min.) 75mg | 82.33±20.21 | 88.29±20.62 | 83.44±32.30 | 0,830 |
| Awakenings | 12.84±3.37@ | 10.92±2.89# | 11.49±3.186@# | **<0,001** |
| Awakenings 50 mg | 12.97±3.85@ | 10.68±3.44 | 11.35±3.59@ | **0,020** |
| Awakenings 75 mg | 12.64±2.57 | 11.37±1.56 | 11.71±2.54£2 | 0,070 |

An improvement in sleep efficiency and in the number of awakenings was observed in patients after the use of the formulations developed.

In short, the study data associated with the data available in scientific literature demonstrate the safety of the developed trazodone formulations and their possible efficacy in sleep disorders, especially with regard to improving sleep quality, as demonstrated by the results obtained in the Pittsburgh Sleep Quality Questionnaire, Athens Insomnia Index, Insomnia Severity Scale, sleep latency, total sleep time, and reduction in the number of awakenings through actigraphy.

Added to this, such innovation comprises the use in the layers containing the active ingredient of an excipient that functions as an antioxidant, which helps with the stability of the drug, considering that, although the active ingredient trazodone is not susceptible to oxidative degradation, when in process, it showed a change in color and in the impurity profile.

Unlike conventional formulations, the formulation and manufacturing process proposed in the present invention present specific characteristics that could induce degradation of the suspended product during industrial production, due to direct contact with oxygen during the manufacturing process, as a result of the use of a micronized active ingredient, in a long process with high oxygen flow, which was addressed by the addition of the antioxidant to the formulation.

Still in the layers containing the active ingredient, the invention consists of providing a wetting agent capable of improving the wettability of the active ingredient in an aqueous carrier by conferring a smaller contact angle between the active ingredient and the liquid, which results in a larger surface area wetted by the carrier.

In short, the developed formulation has technology that enables the release of two doses at different times, representing an innovative treatment concept in which sleep induction is achieved with a low dose, when compared with the available literature, making it possible to release an additional dose during the night so that the patient maintains a longer sleep period and does not wake up and, at the same time, does not experience drowsiness the following morning.

### OBJECTIVES OF THE INVENTION

The main objective of the invention is to provide an oral pharmaceutical form for biphasic release of trazodone. Said pharmaceutical form comprising a core, a coating layer of the active ingredient, a modified-release layer, an immediate-release layer of the active ingredient, and a polymeric layer, the latter being the outermost. Still, a slider is deposited on such pharmaceutical form, which will remain on the surface of the pharmaceutical form.

In addition, it is also one of the objectives of the invention to prepare an oral pharmaceutical form having a biphasic release system of the active pharmaceutical ingredient.

System in which there is an immediate-release of one dose, a latency period in the release, and a second dose release, which may generate a second plasma concentration peak or a plateau in plasma concentration for a certain period of time.

It is also an objective of the invention to provide a medicament in an oral pharmaceutical form for the treatment or prevention of disorders or diseases in an individual, particularly for the treatment of insomnia or improvement of sleep quality.

Another objective of the invention is to provide drug release using a multiparticulate system, which promotes drug release in a specific manner through a pharmaceutical form.

### SUMMARY OF THE INVENTION

The objectives discussed above are, in their entirety, achieved by the oral pharmaceutical form, by the biphasic release system of the active ingredient, by the process for preparing them, and by the therapeutic use of the present invention.

According to the present invention in question, the oral pharmaceutical form comprises a core, a coating layer comprising an active pharmaceutical ingredient, a modified-release layer, an immediate-release layer of the active ingredient, a polymeric layer, and on this outermost layer a slider will be deposited, which will remain on the surface of the pharmaceutical form.

In one embodiment of the invention, the core and the coating layer of the active ingredient form pellets obtained by coating cores with the coating layer.

The term "active pharmaceutical ingredient," API or "active ingredient," as defined in the invention, refers to trazodone or a pharmaceutically acceptable salt, hydrate, solvate, or polymorph thereof, preferably trazodone hydrochloride.

In one embodiment of the invention, said pharmaceutical form is in unit dose form, such as a capsule, a tablet, sachet, sprinkle, suspension, orodispersible tablet, dragee, dispersible tablet.

Further according to the invention, the process for preparing the oral pharmaceutical form comprises the steps of:
(i) preparing a core;
(ii) applying the coating layer comprising the active pharmaceutical ingredient to the core to form pellets coated with the active ingredient;
(iii) applying the modified-release layer to the pellets prepared in (ii); and
(iv) applying the immediate-release layer to the pellets prepared in (iii).

In one embodiment of the invention, said process further comprises:
(v) applying a polymer coating layer to the pellets prepared in (iv).

In another embodiment of the invention, said process comprises depositing a slider on the surface of the oral pharmaceutical form.

In another embodiment of the invention, said process further comprises converting the product into a final pharmaceutical form.

The invention also relates to the use of the oral pharmaceutical form in preparing a medicament for the treatment or prevention of disorders and diseases in an individual.

In one embodiment of the invention, said treatment is the treatment of sleep disorders in patients.

### BRIEF DESCRIPTION OF THE DRAWINGS

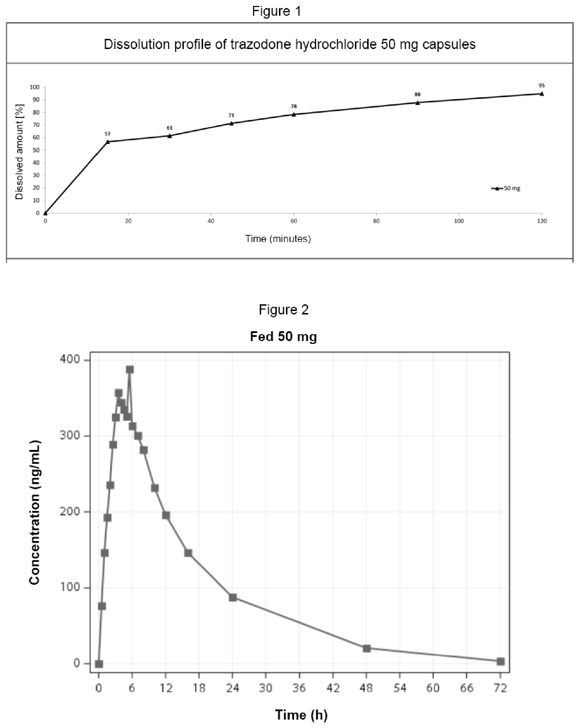

Figure 1 represents the *in vitro* dissolution profile of a first 50 mg trazodone OAD formulation. The dissolution method is carried out in two steps, the first being the acid step, where the dissolution medium is 0.1 M hydrochloric acid, at 37°C, with a medium volume of 500 mL, using the Basket, at 75 rpm, for 60 minutes, and the second step, the buffer step, which uses Potassium Phosphate Buffer pH 6.8 as the medium, at 37°C, with a medium volume of 1000 mL, using the basket at 75 rpm, for 60 minutes. The profile result presented corresponds to the sum of the two steps.
Figure 2 represents the plasma concentration of trazodone derived from a capsule of the first OAD formulation of trazodone 50 mg as a function of time when administered to a human in fed state.

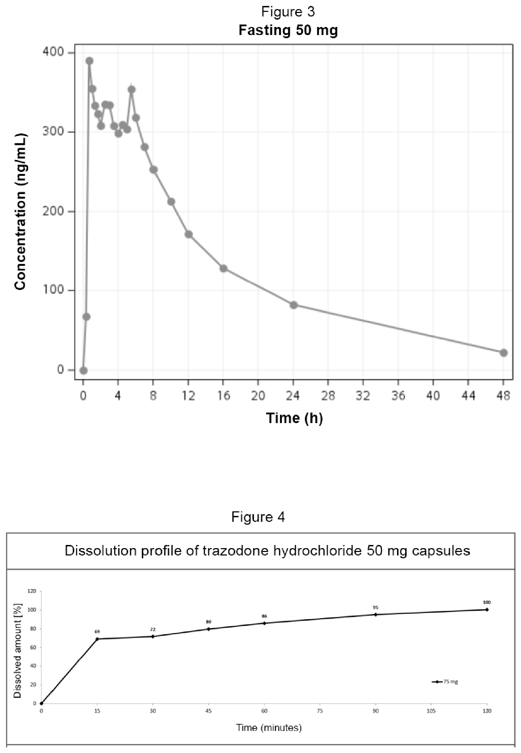

Figure 3 represents the plasma concentration of trazodone derived from a capsule of the first OAD formulation of trazodone 50 mg as a function of time when administered to a human in fasting state.
Figure 4 represents the in *vitro* dissolution profile of a first 75 mg trazodone OAD formulation.

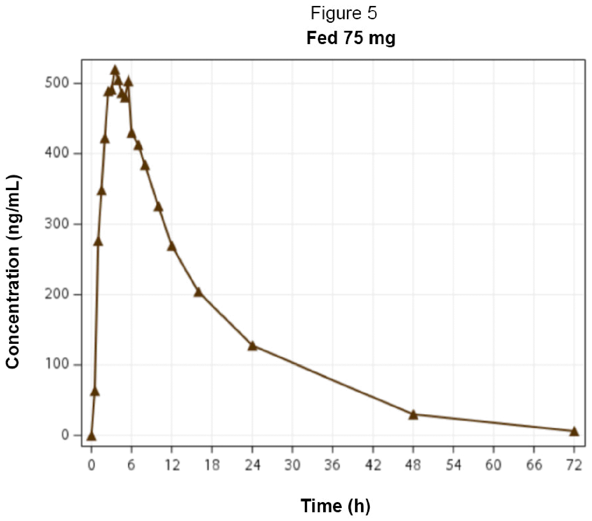

Figure 5 represents the plasma concentration of trazodone derived from a capsule of the first OAD formulation of trazodone 75 mg as a function of time when administered to a human in fed state.

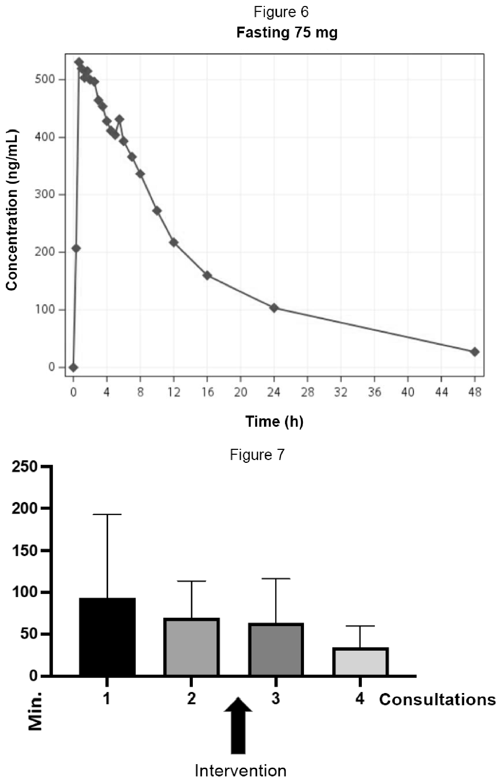

Figure 6 represents the plasma concentration of trazodone derived from a capsule of the first OAD formulation of trazodone 75 mg as a function of time when administered to a human in fasting state.

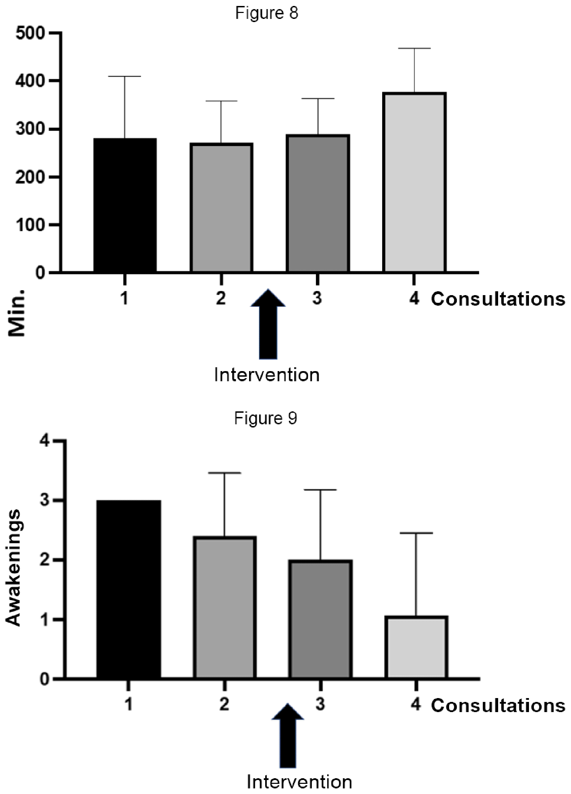

Figure 7 represents the results of the Sleep Latency study, in minutes, demonstrating the patient's condition at the beginning of treatment and after the intervention, which was the administration of the formulation proposed in this patent application. Figure 8 is a graph that represents Total Sleep Time, in minutes, demonstrating the patient's condition at the beginning of treatment and after the intervention, which was the administration of the formulation proposed in this patent application.
Figure 9 represents the Awakenings per Night, demonstrating the patient's condition at the beginning of treatment and after the intervention, which was the administration of the proposed formulation in this patent application.

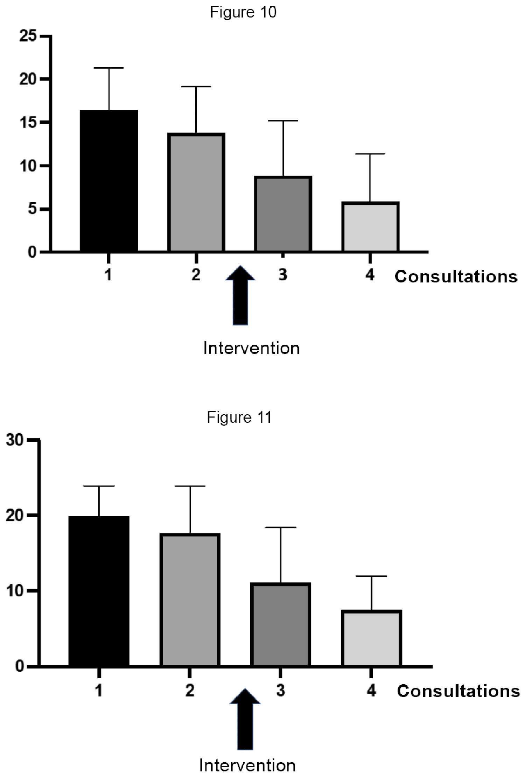

Figure 10 represents the Athens Insomnia Scale, demonstrating the patient's condition at the beginning of treatment and after the intervention, which was the administration of the proposed formulation in this patent application.
Figure 11 represents the Insomnia Severity Index, demonstrating the patient's condition at the beginning of treatment and after the intervention, which was the administration of the proposed formulation in this patent application.

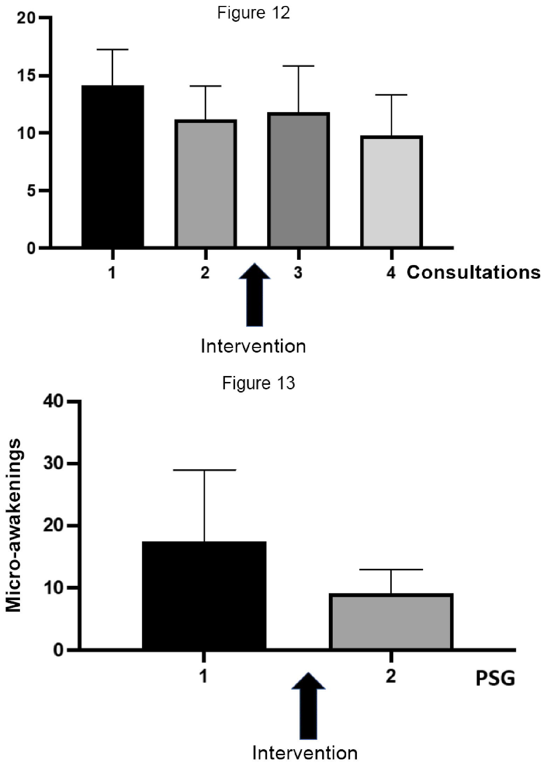

Figure 12 represents sleep quality based on the Pittsburgh Sleep Quality Index, demonstrating the patient's condition at the start of treatment and after the intervention, which was the administration of the formulation proposed in this patent application.
Figure 13 represents the results of polysomnography, evaluating micro-awakenings per night, demonstrating the patient's condition before and after the intervention, which was the administration of the proposed formulation in this patent application.

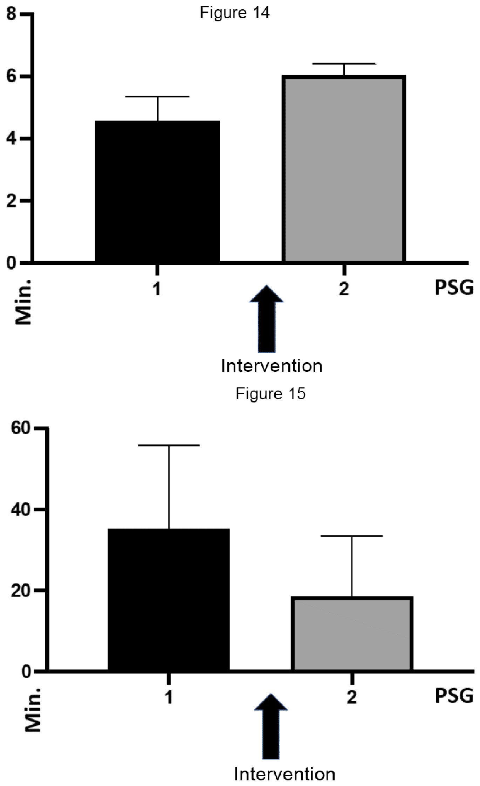

Figure 14 represents the results of polysomnography, evaluating Total Sleep Time, in minutes, demonstrating the patient's condition before and after the intervention, which was the administration of the formulation proposed in this patent application.
Figure 15 represents the results of polysomnography, evaluating wake time, in minutes, demonstrating the patient's condition before and after the intervention, which was the administration of the formulation proposed in this patent application.

### DETAILED DESCRIPTION OF THE INVENTION

In view of the objectives of the present invention, an oral pharmaceutical form for biphasic release of trazodone is disclosed, as well as its therapeutic use and a process for manufacturing said pharmaceutical form.

The oral pharmaceutical form developed comprises:
- a core;
- a coating layer comprising an active pharmaceutical ingredient (API);
- a modified-release layer;
- an immediate-release layer comprising the API;
- a polymeric layer;
- a blending layer.

In one embodiment of the invention, the active pharmaceutical ingredient is trazodone hydrochloride.

In the context of the present invention, the term "core" should be understood as a biocompatible core in which there is substantially no active pharmaceutical ingredient. The core materials may be insoluble or soluble materials.

In one embodiment of the invention, the core is selected from the group comprising glass, biocompatible polymers such as cellulose acetate, methyl methacrylate or other acrylics, nylon, polypropylene, silicone, SBR copolymers and the like, polyethylene glycols such as POLYOX^{®} (Union Carbide) or KLUCEL^{®} (Hercules), cellulose spheres, silicon dioxide spheres, and spherical starch and sugar granules, preferably spherical starch and sugar granules.

In a preferred embodiment of the invention, the core is coated by the coating layer of the active ingredient.

In one embodiment of the invention, the coating layer of the active ingredient is a solution or suspension comprising the API and at least one selected from the group consisting of one or more solvents, a diluent, a wetting agent, a polymer, a slider, an antioxidant, or a combination thereof.

In one embodiment of the invention, the immediate-release layer of the active ingredient is a solution or suspension comprising the API and at least one selected from the group consisting of one or more solvents, a diluent, a wetting agent, a polymer, a slider, an antioxidant, or a combination thereof.

In a particular embodiment of the invention, the coating layer of the active ingredient and the immediate-release layer of the active ingredient have the same or different components/materials, preferably the same.

In one embodiment of the invention, the modified-release layer is a solution or suspension comprising at least one selected from the group consisting of a solvent, a polymer, a slider, a plasticizer, or a combination thereof.

In a particular embodiment of the invention, there is substantially no active pharmaceutical ingredient in the modified-release layer.

In a preferred embodiment of the invention, said oral pharmaceutical form further comprises a polymeric coating layer.

In one embodiment of the invention, the polymer coating layer is a solution or suspension comprising at least one solvent and a polymer, for the purpose of photoprotection of the active ingredient.

In another embodiment of the invention, said oral pharmaceutical form further comprises the deposition of a slider in the final formulation.

In one embodiment of the invention, the blending layer is applied to reduce the electrostatic charge of the pellets, facilitating the encapsulation process, ensuring adequate dosage and content uniformity in the final product. The blending layer may be selected from the group consisting of talc, polyethylene glycol, simethicone, titanium dioxide, magnesium stearate, sodium stearyl fumarate, hydrogenated vegetable oil, carnauba wax, corn starch, magnesium oxide, magnesium silicate, silicon dioxide, calcium phosphate, microcrystalline cellulose, and combinations thereof, preferably talc.

In another embodiment of the invention, said oral pharmaceutical form further comprises conversion into a pharmaceutical form of the final formulation.

In a preferred embodiment of the invention, the solvent is selected from the group comprising purified water, deionized water, distilled water, methanol, ethanol, glycerin, sorbitol, acetone, methyl ethyl ketone, methylene chloride, isopropanol, butylene glycol, benzyl alcohol, dibutyl phthalate, ethyl acetate, ethyl lactate, ethyl oleate, isopropyl myristate, isopropyl palmitate, mineral oil, medium-chain triglycerides, methyl lactate, monoethanolamine, octyldodecanol, polyethylene glycol, propylene carbonate, polyethylene glycol 35 castor oil, pyrrolidone, corn oil, olive oil, peanut oil, cottonseed oil, sunflower oil, canola oil, soybean oil, grapeseed oil, sesame oil, safflower oil, linseed oil, coconut oil, palm oil, triacetin, tricaprylin, triethanolamine, triethyl citrate, triolein, propylene glycol, 2-(2-ethoxyethoxy)ethanol (transcutol), N-methyl-2-pyrrolidone (Pharmasolve), fish oil (omega 3), and combinations thereof, preferably water and ethanol.

In a particular embodiment of the invention, the diluent is selected from the group comprising microcrystalline cellulose, lactose monohydrate, mannitol, xylitol, sucrose, cyclodextrin, calcium carbonate, magnesium carbonate, di- or tribasic calcium phosphate, calcium hydrogen phosphate, pregelatinized starch, low-substituted hydroxypropyl cellulose, alginate, calcium lactate, calcium silicate, calcium sulfate, cellulose acetate, dextran, erythritol, ethylcellulose, fructose, fumaric acid, glyceryl palmitostearate, isomalt, kaolin, lactitol, magnesium carbonate, magnesium oxide, maltodextrin, maltose, polydextrose, sodium chloride, sorbitol, talc, trehalose, and combinations thereof, preferably sucrose.

In a particular embodiment of the invention, the wetting agent is selected from the group comprising polyethylene glycol (PEG), propylene glycol, glycerol, sorbitol, sodium lauryl sulfate, polysorbate 20, polysorbate 40, polysorbate 60, polysorbate 80, sodium docusate, sodium cetearyl sulfate, saponin, and glycerin, and combinations thereof, preferably polysorbate.

In a preferred embodiment of the invention, the antioxidant is selected from the group comprising acetylcysteine, alpha tocopherol, ascorbyl palmitate, butyl hydroxyanisole (BHA), butylated hydroxytoluene (BHT), monothioglycerol, potassium nitrate, sodium ascorbate, sodium formaldehyde sulfoxylate, sodium metabisulfite, sodium bisulfite, vitamin E or a derivative thereof, propyl gallate, disodium edetate (EDTA), diethylenetriamine pentaacetic acid (DTPA), triglycolamate (NT), ascorbic acid, citric acid, erythorbic acid, ethyl oleate, fumaric acid, malic acid, methionine, resveratrol, phosphoric acid, potassium metabisulfite, propionic acid, tert-butylhydroquinone, sodium sulfite, sodium thiosulfate, vitamin E polyethylene glycol succinate, and combinations thereof, preferably propyl gallate.

In a preferred embodiment of the invention, the polymer is selected from the group comprising polyesters such as polylactic acid (PLA), polycaprolactone, poly(lactide-co-glycolide) (PLGA), polyhydroxybutyrate, poly-β-malic acid); polyanhydrides such as poly(adipic anhydride) (PAA), poly(fumaric-co-sebacic) anhydride or (PFA:SA), poly(sebacic anhydride) (PSA); cellulosic polymers such as water-soluble cellulose ethers, such as hypromellose or hydroxypropyl methylcellulose, propylcellulose and/or hydroxypropylcellulose, hydroxyethylcellulose, cellulose acetate, cellulose acetate phthalate, polyvinyl alcohol and its derivatives such as commercially available block copolymers of polyvinyl alcohol and polyethylene glycol as Kollicoat^{©} IR or Kollicoat^{©} Protect, acrylate and methacrylate polymers, such as Eudragit RS 100, RL 100, E100 PO, L100-55, L100, S100, I100, hypromellose (HPMC) and hydroxypropyl methylcellulose-based composition or polyvinyl alcohol obtained under the trade name Opadry^{©} and combinations thereof, preferably hypromellose, or a polymer derived therefrom, and methacrylate copolymer.

In a particular embodiment of the invention, the slider is selected from the group comprising talc, silica, such as colloidal silica, magnesium oxide, powdered cellulose, silicates, such as magnesium silicate, magnesium trisilicate, aluminum magnesium silicates, pyrogenic silica, CAB-O-SIL^{©} M-5P, AEROSIL^{©}, starch, and combinations thereof, preferably talc.

In another embodiment of the invention, the plasticizer is selected from the group consisting of polyethylene glycol, propylene glycol, glycerin, simethicone, triacetin, citric acid esters, such as tributyl citrate, triethyl citrate, sebacic acid esters, such as dibutyl sebacate, phthalic acid esters, such as dimethyl phthalate, mono-, di- and triglycerides of medium-chain fatty acids, castor oil, long-chain fatty acids, and combinations thereof, preferably triethyl citrate.

In one embodiment of the invention, the oral pharmaceutical form is selected from the group comprising a capsule, a tablet, a sachet, a sprinkle, a suspension, an orally disintegrating tablet, a dragee, and a dispersible tablet.

In a preferred embodiment of the invention, the oral pharmaceutical form is a capsule or a tablet.

In an even more preferred embodiment of the invention, the oral pharmaceutical form is a capsule.

In a particular embodiment of the invention, the pharmaceutical form presents the first release with about 5 mg to about 120 mg of the active pharmaceutical ingredient, preferably about 25 mg to about 75 mg of the API, even more preferably about 25 mg to about 50 mg of the API. Further, the same pharmaceutical form presents the second release with about 5 mg to about 120 mg of the active pharmaceutical ingredient, preferably about 25 mg to about 75 mg of the API, even more preferably about 25 mg to about 50 mg of the API.

In one embodiment of the invention, the core is present in the pharmaceutical form in an amount ranging from about 10% to about 60% by weight based on the total weight of said pharmaceutical form, preferably from 20% to 40% by weight.

In one embodiment of the invention, the active pharmaceutical ingredient is present in the coating layer of the active ingredient in an amount ranging from about 1% to about 40% by weight, based on the total weight of said pharmaceutical form, preferably from 5% to about 20% by weight.

In one embodiment of the invention, the active pharmaceutical ingredient is present in the immediate-release layer of the active ingredient in an amount ranging from about 10% to about 80% by weight based on the total weight of the active pharmaceutical ingredient of said pharmaceutical form, preferably between 45 and 65% by weight.

In one embodiment of the invention, the blending layer is present in a proportion ranging from about 0.1 to about 10% by weight of the pharmaceutical form.

In one embodiment of the invention, the process for preparing the pharmaceutical form comprises previously preparing a coating layer of the active ingredient by forming a solution or suspension comprising the API and at least one selected from the group comprising one or more solvents, a diluent, a wetting agent, a polymer, a slider, an antioxidant, or a combination thereof.

In one embodiment of the invention, the process comprises previously preparing a core by preheating said core.

In one embodiment of the invention, the process further comprises applying the coating layer comprising the API to the preheated core to form pellets coated with the active ingredient.

In one embodiment of the invention, the process for preparing the pharmaceutical form comprises previously preparing a modified-release layer by forming a solution or suspension comprising at least one selected from the group consisting of a solvent, a polymer, a slider, a plasticizer, or a combination thereof.

In a particular embodiment of the invention, the process comprises applying the modified-release layer to the pellets previously coated with a first coating layer of the active ingredient.

In one embodiment of the invention, the process for preparing the pharmaceutical form comprises preparing an immediate-release layer of the active ingredient by forming a solution or suspension comprising the API and at least one selected from the group consisting of one or more solvents, a diluent, a wetting agent, a polymer, a slider, an antioxidant, or a combination thereof.

In a preferred embodiment of the invention, the process comprises applying the immediate-release layer of the active ingredient to the pellets previously coated with a first coating layer of the active ingredient and a second modified-release layer.

In another embodiment of the invention, said process further comprises applying a polymeric coating layer to the pellets previously coated with a first coating layer of the active ingredient, a second modified-release layer, and a third immediate-release layer of the active ingredient.

In a particular embodiment of the invention, said process further comprises depositing a slider onto the surface of the pellets previously coated with a first coating layer of the active ingredient, a second modified-release layer, a third immediate-release layer of the active ingredient, and a fourth polymeric coating layer.

In another particular embodiment of the invention, said process further comprises incorporating into a final pharmaceutical form the pellets previously coated with a first coating layer of the active ingredient, a second modified-release layer, a third immediate-release layer of the active ingredient, and a fourth polymeric coating layer, on which the slider is superficially deposited.

In one embodiment of the invention, the process for preparing the pharmaceutical form comprises the following steps:
(i) preparing a core to receive the film;
(ii) applying the coating layer comprising the active pharmaceutical ingredient to the core to form pellets coated with the active ingredient;
(iii) applying the modified-release layer to the pellets prepared in (ii); and
(iv) applying the immediate-release layer to the pellets prepared in (iii).

In one embodiment of the invention, the cores are fed into the fluidized bed chamber and subsequently suspended by a stream of hot air. A polymeric suspension or solution is sprayed onto the cores, producing the formation of a film, which will promote the adhesion of the drug to the core and/or modulate its release from a system formed by the air inlet volume parameters, product temperature, and spray rate. Thus, the steps of the production process are carried out continuously in a single piece of equipment.

In one embodiment of the invention, the pharmaceutical form is for the treatment or prevention of sleep disorders, post-traumatic disorder, depression, and anxiety, preferably for the treatment of insomnia and/or improvement of sleep quality in patients.

The term bioavailability as defined herein refers to the extent and rate at which a drug reaches the circulatory system after administration.

The present invention is described below with reference to the following examples, which are not intended to limit the scope of the present invention in any way.

To enable a more complete understanding of the embodiments described herein, the following examples are presented. It should be understood that these examples are for illustration purposes only and should not be construed as limiting.

### EXAMPLE 1: PHARMACEUTICAL FORM ACCORDING TO THE INVENTION COMPRISING 50 MG OF THE API

In a preferred embodiment, the pharmaceutical form of the present invention comprises the ingredients in Table 3 below.

**TABLE 3 - EXEMPLARY FORMULATION OF THE PHARMACEUTICAL FORM**

| **Step** | **Material Description** | **Function** | **Quantitative** composition | **% w/w** |
|---|---|---|---|---|
| | Trazodone hydrochloride | Ifa | 27,000 mg | 14,282 |
| | Sucrose/starch spheres | Diluent | 70,000 mg | 37,027 |
| | Talc | Slider | 3,500 mg | 1,851 |
| | Propyl gallate | Antioxidant | 0.200 mg | 0,106 |
| Layer of coating of active | Hypromellose 2910 | Polymer | 9,000 mg | 4,761 |
| | Polysorbate 80 | Wetting agent | 1,500 mg | 0,793 |
| | Granular sucrose type k | Diluent | 2,800 mg | 1,481 |
| | Ethyl alcohol (96 gl)* | Solvent | 0.002 mL | - |
| | Purified Water* | Solvent | 0.230 mL | - |
| Layer of release modified | Eudragit I100) | Polymer | 17,000 mg | 8,992 |
| | Talc | Slider | 8,500 mg | 4,496 |
| | Triethyl citrate | Plasticizer | 1,700 mg | 0,899 |
| | Ethyl alcohol (96 gl) | Solvent | 0.200 ml | - |
| | Trazodone hydrochloride | Ifa | 23,000 mg | 12,166 |
| | Talc | Slider | 3,700 mg | 1,957 |
| Layer of release immediate of the active | Propyl gallate | Antioxidant | 0.200 mg | 0,106 |
| | Hypromellose 2910 | Polymer | 8,800 mg | 4,655 |
| | Polysorbate 80 | Wetting agent | 1,500 mg | 0,793 |
| | Granular sucrose type k | Diluent | 2,800 mg | 1,481 |
| | Ethyl alcohol (96 gl)* | Solvent | 0.002 mL | - |
| | Purified Water* | Solvent | 0.230 mL | - |
| Polymeric layer | Opadry white | Polymer | 7,500 mg | 3,967 |
| | Purified Water* | Solvent | 0.040 mL | - |
| | Ethyl alcohol (96 gl)* | Solvent | 0.040 mL | - |
| Blending | Talc | Slider | 0.350 mg | 0,185 |

| | | | | |
|---|---|---|---|---|
| *Evaporates during the process | | | | |

### EXAMPLE 2: Pharmaceutical form according to the invention comprising 75 mg of the API

In a preferred embodiment, the pharmaceutical form of the present invention comprises the ingredients in Table 4 below.

**TABLE 4 - EXEMPLARY FORMULATION OF THE PHARMACEUTICAL FORM**

| | **Material Description** | **Function** | **Quantitative** composition | **% w/w** |
|---|---|---|---|---|
| | Trazodone hydrochloride | Ifa | 23,000 mg | 10,000 |
| | Sucrose/starch spheres | Diluent | 70,000 mg | 30,435 |
| | Talc | Slider | 3,500 mg | 1,522 |
| Layer of coating of active | Propyl gallate | Antioxidant | 0.200 mg | 0,087 |
| | Hypromellose 2910 | Polymer | 7,700 mg | 3,348 |
| | Polysorbate 80 | Wetting agent | 1,400 mg | 0,609 |
| | Granular sucrose type k | Diluent | 2,200 mg | 0,957 |
| | Ethyl alcohol (96 gl)* | Solvent | 0.002 mL | - |
| | Purified Water* | Solvent | 0.230 mL | - |
| | Eudragit L100 | Polymer | 17,000 mg | 7,391 |
| | Talc | Slider | 8,000 mg | 3,478 |
| release modified | Triethyl citrate | Plasticizer | 1,600 mg | 0,696 |
| | Ethyl alcohol (96 gl)* | Solvent | 0.200 ml | - |
| | Trazodone hydrochloride | Ifa | 52,000 mg | 22,609 |
| | Talc | Slider | 7,100 mg | 3,087 |
| Layer of release immediate of the active | Propyl gallate | Antioxidant | 0.350 mg | 0,152 |
| | Hypromellose 2910 | Polymer | 19,500 mg | 8,478 |
| | Polysorbate 80 | Wetting agent | 2,700 mg | 1,174 |
| | Granular sucrose type k | Diluent | 5,000 mg | 2,174 |
| | Ethyl alcohol (96 gl)* | Solvent | 0.002 mL | - |
| | Purified Water* | Solvent | 0.600 mL | - |
| | Opadry white | Polymer | 8,500 mg | 3,696 |
| | Purified Water* | Solvent | 0.060 ml | - |
| | Ethyl alcohol (96 gl)* | Solvent | 0.060 ml | - |
| Blending | Talc | Slider | 0.250 mg | 0,109 |

In a particular embodiment of the invention, the preparation of the pharmaceutical forms comprising the API according to the formulations of Example 1 and Example 2 was carried out by the production processes detailed below.

### PREPARING THE COATING LAYER OF THE ACTIVE INGREDIENT

The process for preparing the pharmaceutical form comprises previously preparing a coating layer of the active ingredient according to the following steps:
(i) dispersing the excipients propyl gallate, polysorbate 80, granular sucrose type K, talc, and the active ingredient in the purified water;
(ii) adding hypromellose to (i) and keeping under stirring until complete solubilization;
(iii) adding ethyl alcohol (96°GL) to (ii) and keeping the active ingredient suspension under stirring throughout the application of the coating layer of the active ingredient.

### APPLYING THE COATING LAYER OF THE ACTIVE INGREDIENT

The process for preparing the pharmaceutical form further comprises applying the coating layer of the active ingredient to a core, according to the following steps:
(i) adding the spheres in a fluidized bed;
(ii) heating the spheres of (i) until they reach a temperature range of 38°C to 46°C;
(iii) applying to the spheres of (ii) the coating layer of the active ingredient that is in the form of a suspension of the active ingredient, maintaining the system temperature between 38°C and 45°C; and
(iv) forming pellets of cores coated with a first coating layer of the active ingredient;
(v) drying the pellets coated in (iv) in the fluidized bed.

### PREPARING THE MODIFIED-RELEASE LAYER

The process for preparing the pharmaceutical form comprises previously preparing a modified-release layer according to the following steps:
(i) dispersing the excipients talc, triethyl citrate, and methacrylic acid copolymer (Eudragit L 100) in ethyl alcohol (96°GL) and maintain under stirring, thereby forming a suspension that constitutes the modified-release layer of the present pharmaceutical form.

### APPLYING THE MODIFIED-RELEASE LAYER

The process for preparing the pharmaceutical form further comprises applying the modified-release layer as a second layer to the pellets of cores coated with a first coating layer of the active ingredient. The application of the modified-release layer was carried out according to the following steps:
(i) adding, in a fluidized bed, pellets coated with the first coating layer of the active ingredient;
(ii) heating the pellets of (i) until they reach a temperature range of 30°C to 35°C;
(iii) applying the modified-release layer to the pellets of (ii), maintaining the system temperature between 28°C and 35°C; and
(iv) forming pellets of cores coated with a first coating layer of the active ingredient and a second modified-release layer.
(v) drying the pellets coated in (iv) in the fluidized bed.

The process comprises, after drying the pellets coated with the second modified-release layer, sieving the pellets.

### PREPARING THE IMMEDIATE-RELEASE LAYER OF THE ACTIVE INGREDIENT

The process for preparing the pharmaceutical form comprises first preparing an immediate-release layer of the active ingredient according to the following steps:
(i) Dispersing/dissolving the excipients propyl gallate, polysorbate 80, granular sucrose type K, talc, and the active ingredient in the purified water;
(ii) adding hypromellose to (i) and keeping under stirring until complete solubilization;
(iii) adding ethyl alcohol (96°GL) to (ii) and keeping the suspension of the active ingredient under stirring throughout the application of the immediate-release layer of the active ingredient.

### APPLYING THE IMMEDIATE-RELEASE LAYER OF THE ACTIVE INGREDIENT

The process for preparing the pharmaceutical form further comprises applying the immediate-release layer of the active ingredient as a third layer to the pellets of cores coated with a first layer of active ingredient coating and a second modified-release layer. The application of the immediate-release layer of the active ingredient was carried out according to the following steps:
(i) adding, in a fluidized bed, pellets coated with the first coating layer of the active ingredient and second modified-release layer;
(ii) heating, in the fluidized bed, the pellets from (i) until they reach a temperature range of 42°C to 46°C;
(iii) applying, in the fluidized bed, to the pellets of (ii), the immediate-release layer of the active ingredient, maintaining the system temperature between 38°C and 45°C; and
(iv) forming pellets from cores coated with a first coating layer of the active ingredient, a second modified-release layer, and a third immediate-release layer of the active ingredient.
(v) drying the pellets coated in (iv) in the fluidized bed.

The process comprises, after drying the pellets coated with the third immediate-release layer of the active ingredient, sieving the pellets.

### PREPARING THE POLYMERIC COATING LAYER

The process for preparing the pharmaceutical form comprises previously preparing a polymeric coating layer according to the following steps:
(i) preparing a hydroalcoholic solution;
(ii) dispersing the Opadry White polymer in the solution of (i) and keeping under stirring, thus forming a dispersion that constitutes the polymeric coating layer of the present pharmaceutical form.

### APPLYING THE POLYMERIC COATING LAYER

The process for preparing the pharmaceutical form further comprises applying the polymeric coating layer as a fourth layer to the pellets of cores coated with a first coating layer of the active ingredient, a second modified-release layer, and a third immediate-release layer of the active ingredient. The application of the polymer coating layer was carried out according to the following steps:
(i) adding, in a fluidized bed, pellets coated with the third immediate-release layer of the active ingredient;
(ii) heating, in the fluidized bed, the pellets of (i) until they reach a temperature range of 40°C to 45°C;
(iii) applying, in the fluidized bed, to the pellets of (ii), the polymeric coating layer, maintaining the system temperature between 38°C and 45°C; and
(iv) forming pellets from cores coated with a first coating layer of the active ingredient, a second modified-release layer, a third immediate-release layer of the active ingredient, and a fourth polymeric coating layer.
(v) drying the pellets coated in (iv) in the fluidized bed.

The process comprises, after drying the pellets coated with the fourth layer of polymeric coating, performing sieve classification of the pellets.

The process also comprises blending the pellets coated with the fourth polymeric coating layer, so as to carry out the deposition of talc on the surface of the pellets and a physical interaction.

The process further comprises transforming the pellets coated with the fourth polymer coating layer into a pharmaceutical form.

Having described the embodiments of the pharmaceutical form, use, and manufacturing process, it should be understood that the scope of protection in question also encompasses undescribed variations, but derived or equivalent.

## Claims

1. An oral pharmaceutical form **CHARACTERIZED by** comprising: a core;
a coating layer comprising an active pharmaceutical ingredient (API);
a modified-release layer;
an immediate-release layer comprising the API;
in which the active pharmaceutical ingredient is trazodone or an acceptable salt, and a polymeric layer.

2. The pharmaceutical form according to claim 1, **CHARACTERIZED in that** the core is coated by the coating layer of the active ingredient.

3. The pharmaceutical form according to claim 1 or 2, **CHARACTERIZED in that** it promotes two peaks of API release, considering the interval between releases of 1 to 6 hours.

4. The pharmaceutical form according to claim 1 or 2, **CHARACTERIZED in that** the coating layer of the active ingredient and the immediate-release layer of the active ingredient have the same concentration of the API.

5. The pharmaceutical form according to claim 1 or 2, **CHARACTERIZED in that** the coating layer of the active ingredient and the immediate-release layer of the active ingredient have different concentrations of the API.

6. The pharmaceutical form according to any one of claims 1 to 5, **CHARACTERIZED in that** the API is trazodone hydrochloride.

7. The pharmaceutical form according to any one of claims 1 to 6, **CHARACTERIZED in that** the coating layer of the active ingredient is a suspension comprising the API and at least one excipient selected from the group comprising one or more solvents, a diluent, a wetting agent, a polymer, a slider, an antioxidant, or a combination thereof.

8. The pharmaceutical form according to any one of claims 1 to 7, **CHARACTERIZED in that** the immediate-release layer of the active ingredient is a suspension comprising the API and at least one excipient selected from the group comprising one or more solvents, a diluent, a wetting agent, a polymer, a slider, an antioxidant, or a combination thereof.

9. The pharmaceutical form according to claim 7 or 8, **CHARACTERIZED in that** the coating layer of the active ingredient and the immediate-release layer of the active ingredient have the same components/materials.

10. The pharmaceutical form according to any one of claims 1 to 9, **CHARACTERIZED in that** the modified-release layer is a solution or suspension, comprising at least one selected from the group consisting of a solvent, a polymer, a slider, a plasticizer, or a combination thereof.

11. The pharmaceutical form according to any one of claims 7 to 10, **CHARACTERIZED in that**:
(i) the solvent is selected from the group comprising purified water, deionized water, distilled water, methanol, ethanol, glycerin, sorbitol, acetone, methyl ethyl ketone, methylene chloride, isopropanol, butylene glycol, benzyl alcohol, dibutyl phthalate, ethyl acetate, ethyl lactate, ethyl oleate, isopropyl myristate, isopropyl palmitate, mineral oil, medium-chain triglycerides, methyl lactate, monoethanolamine, octyldodecanol, polyethylene glycol, propylene carbonate, polyethylene glycol 35 castor oil, pyrrolidone, corn oil, olive oil, peanut oil, cottonseed oil, sunflower oil, canola oil, soybean oil, grapeseed oil, sesame oil, safflower oil, linseed oil, coconut oil, palm oil, triacetin, tricaprylin, triethanolamine, triethyl citrate, triolein, propylene glycol, 2-(2-ethoxyethoxy)ethanol (transcutol), N-methyl-2-pyrrolidone (Pharmasolve), fish oil (omega 3), and combinations thereof;
(ii) the diluent is selected from the group comprising microcrystalline cellulose, lactose monohydrate, mannitol, xylitol, sucrose, cyclodextrin, calcium carbonate, magnesium carbonate, di- or tribasic calcium phosphate, calcium hydrogen phosphate, pregelatinized starch, low-substituted hydroxypropyl cellulose, alginate, calcium lactate, calcium silicate, calcium sulfate, cellulose acetate, dextrates, erythritol, ethylcellulose, fructose, fumaric acid, glyceryl palmitostearate, isomalt, kaolin, lactitol, magnesium carbonate, magnesium oxide, maltodextrin, maltose, polydextrose, sodium chloride, sorbitol, talc, trehalose, and combinations thereof;
(iii) the wetting agent is selected from the group comprising polyethylene glycol (PEG), propylene glycol, glycerol, sorbitol, sodium lauryl sulfate, polysorbate 20, polysorbate 40, polysorbate 60, polysorbate 80, sodium docusate, sodium cetearyl sulfate, saponin, and glycerin, and combinations thereof;
(iv) the antioxidant is selected from the group consisting of acetylcysteine, alpha tocopherol, ascorbyl palmitate, butyl hydroxyanisole (BHA), butyl hydroxytoluene (BHT), monothioglycerol, potassium nitrate, sodium ascorbate, sodium formaldehyde sulfoxylate, sodium metabisulfite, sodium bisulfite, vitamin E or a derivative thereof, propyl gallate, disodium edetate (EDTA), diethylenetriamine pentaacetic acid (DTPA), triglycolamate (NT), ascorbic acid, citric acid, erythorbic acid, ethyl oleate, fumaric acid, malic acid, methionine, resveratrol, phosphoric acid, potassium metabisulfite, propionic acid, tert-butylhydroquinone, sodium sulfite, sodium thiosulfate, vitamin E polyethylene glycol succinate and combinations thereof;
(v) the polymer is selected from the group comprising polyesters such as polylactic acid (PLA), polycaprolactone, polylactide-co-glycolide (PLGA), polyhydroxybutyrate, polyβ-malic acid); polyanhydrides such as poly(adipic)anhydride (PAA), poly(fumaric-co-sebacic)anhydride or (PFA:SA), poly(sebacic)anhydride (PSA); cellulosic polymers such as water-soluble cellulose ethers, such as hypromellose or hydroxypropyl methylcellulose, propylcellulose, hydroxyethylcellulose, cellulose acetate, cellulose acetate phthalate, polyvinyl alcohol and derivatives thereof such as block copolymers of polyvinyl alcohol and polyethylene glycol, acrylate and methacrylate polymers, hypromellose (HPMC) and hydroxypropyl methylcellulose-based composition or polyvinyl alcohol and combinations thereof;
(vi) the slider is selected from the group comprising talc, silica, such as colloidal silica, magnesium oxide, powdered cellulose, silicates, such as magnesium silicate, magnesium trisilicate, aluminum magnesium silicates, pyrogenic silica, starch, and combinations thereof; and
(vii) the plasticizer is selected from the group comprising polyethylene glycol, propylene glycol, glycerin, simethicone, triacetin, citric acid esters, such as tributyl citrate, triethyl citrate, sebacic acid esters, such as dibutyl sebacate, phthalic acid esters, such as dimethyl phthalate, mono-, di- and triglycerides of medium-chain fatty acids, castor oil, long-chain fatty acids, and combinations thereof.

12. The pharmaceutical form according to any one of claims 1 to 11, **CHARACTERIZED in that** the oral pharmaceutical form further comprises a polymeric coating layer and/or incorporation into a final formulation.

13. The pharmaceutical form according to any one of claims 1 to 12, **CHARACTERIZED in that** the amount of the API present in the coating layer of the active ingredient comprises from 25 to 60% (w/w) of the total amount of API present in the formulation.

14. The pharmaceutical form according to any one of claims 1 to 12, **CHARACTERIZED in that** the amount of the API, present in the immediate-release layer of the active ingredient, comprises from 40 to 70% (w/w) of the total amount of the API present in the formulation.

15. The pharmaceutical form according to any one of claims 1 to 14, **CHARACTERIZED by** comprising 20 to 100 mg of trazodone hydrochloride.

16. The pharmaceutical form according to any one of claims 1 to 15, **CHARACTERIZED in that** the pharmaceutical form has a pharmaceutical form selected from the group comprising a capsule, a tablet, sachet, sprinkle, suspension, orodispersible tablet, dragee, dispersible tablet.

17. The pharmaceutical form according to any one of claims 1 to 16, **CHARACTERIZED in that** it provides a controlled-release system of the API that promotes the biphasic release of trazodone.

18. The pharmaceutical form according to any one of claims 1 to 17, **CHARACTERIZED in that** the pharmaceutical form is composed of a multiparticulate system.

19. A process for preparing the pharmaceutical form as defined in any one of claims 1 to 18, **CHARACTERIZED by** comprising the following steps:
(i) preparing a core;
(ii) applying the coating layer comprising the active pharmaceutical ingredient to the core to form pellets coated with the active ingredient;
(iii) applying the modified-release layer to the pellets prepared in (ii);
(iv) applying the immediate-release layer to the pellets prepared in (iii) and,
(v) applying the polymeric layer to the pellets prepared in (iv).
(vi) incorporation of the multiparticulate system into a pharmaceutical form.

20. Use of the pharmaceutical form as defined in any one of claims 1 to 18, **CHARACTERIZED in that** it is in the preparation of a medicament for the treatment or prevention of insomnia in an individual.

21. The use according to claim 20, **CHARACTERIZED in that** the treatment or prevention of insomnia consists of improving sleep quality, increasing total sleep time, decreasing sleep latency, reducing nighttime awakenings, improving total sleep efficiency, treating or preventing depression, comorbid insomnia, primary and/or secondary insomnia, reducing the severity of insomnia, inducing and/or maintaining sleep.
